# EUROPEAN PATENT APPLICATION

(11) **EP 3 643 307 A1**
(43) Date of publication of application: **29.04.2020**
(21) Application number: 19209255.9
(22) Date of filing: 12.05.2016
(51) Int. Cl.: A61K 31/337, A61P 35/00

(54) **CABAZITAXEL AND ITS USE FOR TREATING CANCER**

(30) Priority: 13.05.2015 EP 15305720
(62) Divisional of application: 16721844.5
(71) Applicant: Sanofi Mature IP, 75008 Paris (FR)
(72) Inventor: CHADJAA, Mustapha, 75008 PARIS (FR); DEDIEU, Jean-François, 75008 PARIS (FR); GUPTA, Sunil, BRIDGEWATER, NJ New Jersey 08807 (US); SELLAMI, Dalila, CHESTER, NJ New Jersey 07930 (US); SEMIOND, Dorothée, BRIDGEWATER, NJ New Jersey 08807 (US)
(74) Representative: Sanofi

(57) **Abstract**

The present invention relates to the compound having the following formula (I): 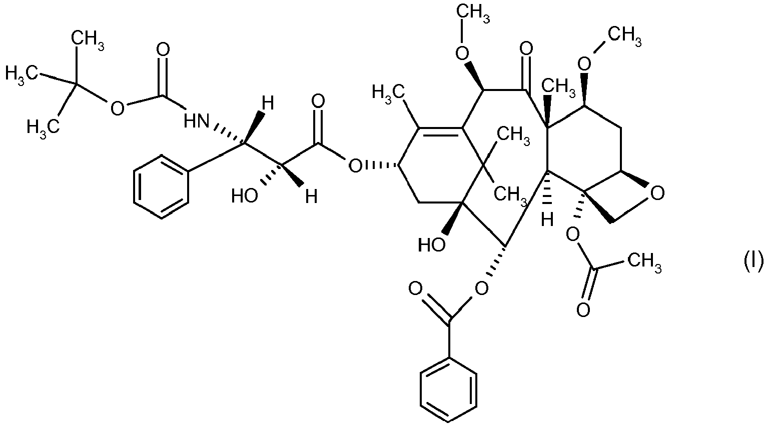 which may be in base form or in the form of a hydrate or a solvate,
for its use as a medicament in the treatment of cancer in patients with hepatic impairment.

## Description

### FIELD OF THE INVENTION

The present invention concerns the use of cabazitaxel for treating cancer in patients with hepatic impairment. It also concerns a method of managing the risk of hepatic impairment to allow an effective and safe use of cabazitaxel in the treatment of cancer.

### BACKGROUND OF THE INVENTION

Cabazitaxel is a semi-synthetic derivative of the natural taxoids 10-deacetylbaccatin III with potential antineoplastic activity. Cabazitaxel binds to and stabilizes tubulin, resulting in the inhibition of microtubule depolymerization and tumor cell proliferation. Unlike other taxane compounds, this agent is active in tumor models poorly or not sensitive to chemotherapy, including taxanes. In addition, cabazitaxel penetrates the blood-brain barrier.

Cabazitaxel has been developed and is registered under the trademark Jevtana ® for the treatment of patients with hormone refractory metastatic prostate cancer (HRPC) previously treated with a docetaxel containing regimen, in combination with prednisone or prednisolone. The recommended dose is 25 mg/m² administered as a 1-hour intravenous infusion every 3 weeks in combination with oral prednisone or prednisolone 10 mg administered daily throughout cabazitaxel treatment. It is available in vials of concentrate (sterile concentrate) and solvent for solution for infusion (60 mg).

It has been observed that the use of cabazitaxel for the treatment of cancer, for example for the treatment of prostate cancer, may provoke some adverse reactions which may be fatal for the patients, notably for patients presenting hepatic impairment.

In the prescribing information as published for Jevtana in the US at its date of launch in June 2010, the following warnings and precautions for use were given: "No dedicated hepatic impairment trial for JEVTANA has been conducted. Patients with impaired hepatic function (total bilirubin ≥ 1 x ULN, or AST and/or ALT ≥ 1.5 x ULN) were excluded from the randomized clinical trial. Cabazitaxel is extensively metabolized in the liver, and hepatic impairment is likely to increase cabazitaxel concentrations. Hepatic impairment increases the risk of severe and life-threatening complications in patients receiving other drugs belonging to the same class as JEVTANA. JEVTANA should not be given to patients with hepatic impairment (total bilirubin ≥ 1 x ULN, or AST and/or ALT ≥ 1.5 x ULN)." A similar wording is included in the prescribing information for Jevtana in all the countries where the product is marketed.

Thus there is a need to find a safe treatment for cancer patients presenting hepatic impairment.

The invention answers that need by providing safe dosage regimens of cabazitaxel or recommendation not to use cabazitaxel depending on the degree of hepatic impairment in the treatment of cancer in patients with varying degrees of hepatic impairment.

### SUMMARY OF THE INVENTION

The present invention relates to the compound having the following formula (I): 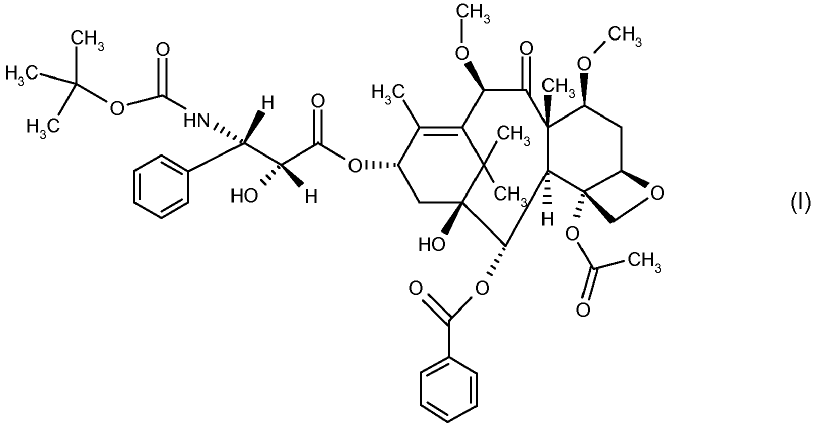 which may be in base form or in the form of a hydrate or a solvate,
for its use as a medicament in the treatment of cancer in patients with hepatic impairment where the administered standard dose of 25 mg/m² of cabazitaxel is reduced or where the administration of cabazitaxel is stopped or to be avoided.

The present invention also relates to a method for treating cancer in a patient with hepatic impairment in need of treatment, said method comprising administering a reduced dose of the compound of formula (I) as defined above, which may be in base form or in the form of a hydrate or a solvate, to said patient, wherein said reduced dose is less than the standard dose of 25 mg/m² of cabazitaxel.

The present invention also relates to a method of managing the risk of hepatic impairment, to allow an effective and safe use of the compound of formula (I) as defined above, in the treatment of patients treated for cancer, said method comprising the following steps:
a) initially check the patients suffering from cancer for any hepatic impairment;
b) if any hepatic impairment is detected, then the administered standard dose of 25 mg/m² of cabazitaxel should be reduced or the administration of cabazitaxel should be stopped or to be avoided;
c) optionally if after a diagnosis of hepatic impairment is excluded, treatment with the compound of formula (I) should be reinitiated, patients should be closely monitored.

The present invention relates to patients with hepatic impairment that may be mild, moderate or severe.

In one aspect of the invention, the cancer to be treated may be a solid tumor. In another aspect, the cancer may be an advanced, measurable or non-measurable, non-hematological cancer. In another aspect, the cancer may be one that is either refractory to standard therapy or for which no standard therapy exists. The cancer may be for example a castration resistant or hormone-refractory metastatic prostate cancer.

In one aspect of the invention, the compound of formula (I) is in the form of an acetone solvate. This acetone solvate may contain between 5% and 8%, and preferably between 5% and 7%, by weight of acetone.

In some aspects of the invention, cabazitaxel may be administered by intravenous infusion at a dose of between 15 and 25 mg/m², this administration cycle of the antitumour agent being repeated at an interval of 3 weeks between each cabazitaxel administration, which interval may be prolonged by 1 to 2 weeks depending on the tolerance to the preceding cabazitaxel administration.

In one aspect of the invention, the administration of the compound of formula (I) is repeated as a new cycle every 3 weeks.

In one aspect of the invention, the reduced and recommended dose for patients with mild hepatic impairment is 20 mg/m².

In one aspect of the invention, the reduced and maximum tolerated dose for patients with moderate hepatic impairment is 15 mg/m².

In one aspect of the invention, the administration of cabazitaxel in patients with mild hepatic impairment may have to be stopped.

In one aspect of the invention, the administration of cabazitaxel in patients with severe hepatic impairment is contraindicated and thus has to be avoided.

### DETAILED DESCRIPTION OF THE INVENTION

### Definitions

The term "patient," as used herein, includes both human and animals. In one embodiment, a patient is a human.

The term "hepatic impairment" as used herein, is an abnormal hepatic function that is to say a reduced liver function.
A normal hepatic function is defined as a total bilirubin level ≤ upper limit of normal [ULN] and by an aspartate aminotransferase level [AST] ≤ ULN.
Abnormal hepatic functions are:
- mild hepatic impairment (total bilirubin >1 to ≤1.5xULN or AST >1.5xULN),
- moderate hepatic impairment (total bilirubin >1.5x to ≤3.0xULN; AST=any),
- severe hepatic impairment (total bilirubin >3.0x - 10xULN; AST=any).

The term "cabazitaxel" corresponds to the compound belonging to the taxoid family of formula (I):

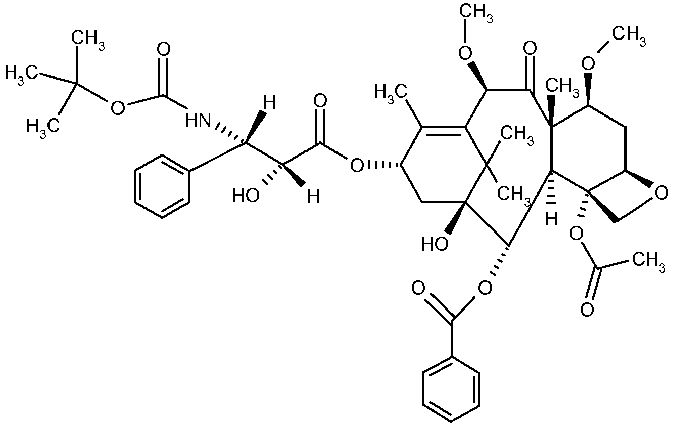

The chemical name of cabazitaxel is 4α-acetoxy-2α-benzoyloxy-5β,20-epoxy-1β-hydroxy-7β,10β-dimethoxy-9-oxo-11-taxen-13α-yl(2R,3S)-3-*tert*-butoxycarbonyl-amino-2-hydroxy-3-phenylpropionate. Cabazitaxel is synonymously known as (2α,5β,7β,10β,13α)-4-acetoxy-13-({(2R,3S)-3-[(tertbutoxycarbonyl)amino]-2-hydroxy-3-phenylpropanoyl}oxy)-1-hydroxy-7,10-dimethoxy-9-oxo-5,20-epoxytax-11-en-2-yl benzoate.

This compound and a preparative method thereof is described in WO 96/30355, EP 0 817 779 B1 and US 5 847 170, which are hereby incorporated herein by reference. Cabazitaxel may be administered in base form (cf. above formula), or in the form of a hydrate. It may also be a solvate, i.e. a molecular complex characterized by the incorporation of the crystallization solvent into the crystal of the molecule of the active principle (see in this respect page 1276 of J. Pharm. Sci. 1975, 64(8), 1269-1288). In particular, it may be an acetone solvate, and, more particularly, may be the solvate described in WO 2005/028462. It may be an acetone solvate of cabazitaxel containing between 5% and 8% and preferably between 5% and 7% by weight of acetone (% means content of acetone/content of acetone+cabazitaxel x 100). An average value of the acetone content is 7%, which approximately represents the acetone stoichiometry, which is 6.5% for a solvate containing one molecule of acetone. The procedure described below allows the preparation of an acetone solvate of cabazitaxel:
940 ml of purified water are added at 20±5°C (room temperature) to a solution of 207 g of 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenyl-propionate at about 92% by weight in about 2 litres of acetone, followed by seeding with a suspension of 2 g of 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate isolated from acetone/water in a mixture of 20 ml of water and 20 ml of acetone. The resulting mixture is stirred for about 10 to 22 hours, and 1.5 litres of purified water are added over 4 to 5 hours. This mixture is stirred for 60 to 90 minutes, and the suspension is then filtered under reduced pressure. The cake is washed on the filter with a solution prepared from 450 ml of acetone and 550 ml of purified water, and then oven-dried at 55°C under reduced pressure (0.7 kPa) for 4 hours. 197 g of 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate acetone containing 0.1% water and 7.2% acetone (theoretical amount: 6.5% for a stoichiometric solvate) are obtained.

One aspect of the present invention relates to the compound having the following formula (I): 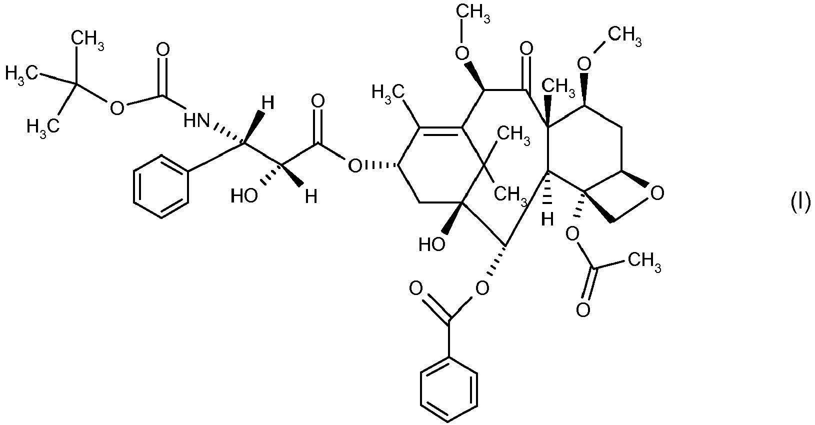 which may be in base form or in the form of a hydrate or a solvate,
for its use as a medicament in the treatment of cancer in patients with hepatic impairment where the administered standard dose of 25 mg/m² of cabazitaxel is reduced or where the administration of cabazitaxel is stopped or to be avoided.

The present invention also relates to a method for treating cancer in a patient with hepatic impairment, said method comprising the administration of the compound of formula (I) as defined above, which may be in base form or in the form of a hydrate or a solvate, where the administered standard dose of 25 mg/m² of cabazitaxel is reduced or where the administration of cabazitaxel is stopped or to be avoided.

The present invention also relates to a method of managing the risk of hepatic impairment, to allow an effective and safe use of the compound of formula (I) as defined above, in the treatment of patients treated for cancer, said method comprising the following steps:
d) initially check the patients suffering from cancer for any hepatic impairment;
e) if any hepatic impairment is detected, then the administered standard dose of 25 mg/m² of cabazitaxel should be reduced or the administration of cabazitaxel should be stopped or to be avoided;
f) optionally if after a diagnosis of hepatic impairment is excluded, treatment with the compound of formula (I) should be reinitiated, patients should be closely monitored.

The present invention relates to patients with hepatic impairment that may be mild, moderate or severe.

In one aspect of the invention, the cancer to be treated may be a solid tumor. In another aspect, the cancer may be an advanced, measurable or non-measurable, non-hematological cancer. In another aspect, the cancer may be one that is either refractory to standard therapy or for which no standard therapy exists. The cancer may be for example a castration resistant or hormone-refractory metastatic prostate cancer.

One aspect of the invention is a method of treating castration resistant or hormone-refractory metastatic prostate cancer in a patient in need thereof, said method comprising administering a reduced dose of the compound of formula (I) as defined above, which may be in base form or in the form of a hydrate or a solvate, to said patient, wherein said patient has hepatic impairment and wherein said reduced dose is less than the standard dose of 25 mg/m² of cabazitaxel.

In one aspect of the invention, the compound of formula (I) is in the form of an acetone solvate. This acetone solvate may contain between 5% and 8%, and preferably between 5% and 7%, by weight of acetone.

In some aspects of the invention, cabazitaxel may be administered by intravenous infusion at a dose of between 15 and 25 mg/m², this administration cycle of the antitumour agent being repeated at an interval of 3 weeks between each cabazitaxel administration, which interval may be prolonged by 1 to 2 weeks depending on the tolerance to the preceding cabazitaxel administration.

In one aspect of the invention, the administration of the compound of formula (I) is repeated as a new cycle every 3 weeks.

In one aspect of the invention, the reduced and recommended dose for patients with mild hepatic impairment is 20 mg/m².

In one aspect of the invention, the reduced and maximum tolerated dose for patients with moderate hepatic impairment is 15 mg/m².

In one aspect of the invention, the administration of cabazitaxel in patients with mild hepatic impairment may have to be stopped.

In one aspect of the invention, the administration of cabazitaxel in patients with severe hepatic impairment is contraindicated and thus has to be avoided. Cabazitaxel may be administered parenterally, such as via intravenous administration. A galenical form of cabazitaxel suitable for administration by intravenous infusion is that in which the cabazitaxel is dissolved in water in the presence of excipients chosen from surfactants, cosolvents, glucose or sodium chloride, etc. For example, a galenical form of cabazitaxel may be prepared by diluting a premix solution of cabazitaxel contained in a sterile vial (80 mg of cabazitaxel + 2 ml of solvent + Polysorbate 80) with a sterile vial containing a solution of 6 ml of water and ethanol (13% by weight of 95% ethanol) in order to obtain 8 ml of a solution ready to be rediluted in a perfusion bag. The concentration of cabazitaxel in this ready-to-redilute solution is about 10 mg/ml. The perfusion is then prepared by injecting the appropriate amount of this ready-to-redilute solution into the perfusion bag containing water and glucose (about 5%) or sodium chloride (about 0.9%).

The present invention is based on the monitoring of the patient concerning the hepatic impairment as mentioned above. Indeed, if the patient to be treated experiences any of these hepatic impairments before or during the administration cycle, then the cancer treatment has to be adapted or discontinued. If the hepatic impairment is resolved, the cancer treatment may be continued. Depending on the patient's conditions, the cancer treatment may also be stopped.

Cabazitaxel is administered repeatedly according to a protocol that depends on the patient to be treated (age, weight, treatment history, etc.), which can be determined by a skilled physician. In one aspect of the invention, cabazitaxel is administered by perfusion to the patient according to an intermittent program with an interval between each administration of 3 weeks, which may be prolonged by 1 to 2 weeks depending on the tolerance to the preceding administration.

In some aspects of the invention, the patient to be treated has prostate cancer that is resistant to hormone therapy (i.e., hormone refractory) and has previously been treated with docetaxel. In some aspects, the patient has prostate cancer that progressed during or after treatment with docetaxel. In some aspects, the patient was previously treated with at least 225 mg/m² cumulative dose of docetaxel. In a particular aspect, the patient showed progression of their disease in the six months following hormone therapy or during docetaxel treatment or after docetaxel treatment. In another particular aspect, the patient showed progression of their disease in the three months following hormone therapy or after docetaxel treatment.

In some aspects of the invention, cabazitaxel is administered to a patient with castration resistant or hormone-refractory metastatic prostate cancer in combination with a corticoid, such as prednisone or prednisolone. The corticoid is preferably administered at a daily dose of 10 mg orally.

In some aspects, the treatment does not include patients for whom:
- Eastern Cooperative Oncology Group (ECOG) performance status (PS) >2
- Life expectancy <3 months
- Need for a major surgical procedure or radiation therapy during the study
- Evidence of another active malignancy
- Prior chemotherapy, other investigational drug, biological therapy, targeted non-cytotoxic therapy and radiotherapy within 3 weeks prior to registration
- Patients with known history of Gilbert's syndrome
- Prior treatment with Cabazitaxel and a history of severe (Grade ≥3) hypersensitivity to taxanes, polysorbate-80, or to compounds with similar chemical structures.

The present invention also relates to a method of providing the compound of formula (I) as defined above, which may be in base form or in the form of a hydrate or a solvate, wherein said compound is provided along with information indicating that it is useful for treating patients with cancer, for example castration resistant or hormone-refractory metastatic prostate cancer, said patients being at risk of developing hepatic impairment.

According to an embodiment, the information comprises printed matter, preferably a label, that advises that the compound of formula (I) is useful for treating patients suffering from cancer, for example castration resistant or hormone-refractory metastatic prostate cancer, said patients being at risk of developing hepatic impairment.

The present invention also relates to a method of promoting the use of the compound of formula (I) as defined above, the method comprising the step of conveying to a recipient at least one message selected from the group consisting of:
(a) the reduced and recommended dose for patients with mild hepatic impairment is 20 mg/m2.
(b) the reduced and maximum tolerated dose for patients with moderate hepatic impairment is 15 mg/m2.
(c) the administration of cabazitaxel in patients with mild hepatic impairment may have to be stopped.
(d) the administration of cabazitaxel in patients with severe hepatic impairment has to be avoided.

The present invention also relates to a method of promoting the use of the compound of formula (I) as defined above, the method comprising the step of conveying to a recipient at least one message selected from the group consisting of:
(a) Patients with mild hepatic impairment (total bilirubin >1 to 1.5 x Upper Limit of Normal (ULN) or AST >1.5 x ULN), should have cabazitaxel dose reduced to 20 mg/m2. Administration of cabazitaxel to patients with mild hepatic impairment should be undertaken with caution and close monitoring of safety;
(b) Limited efficacy data for cabazitaxel at 15 mg/m 2, the maximum tolerated dose in patients with moderate hepatic impairment (total bilirubin >1.5 to < 3 x ULN) are available to recommend this dose in this population;
(c) cabazitaxel should not be given to patients with severe hepatic impairment (total bilirubin >3 xULN).

The present invention also relates to a method of promoting the use of the compound of formula (I) as defined above, the method comprising the step of conveying to a recipient at least one message selected from the group consisting of:
(a) Patients with mild hepatic impairment (total bilirubin >1 to ≤1.5 x Upper Limit of Normal (ULN) or AST >1.5 x ULN), should have cabazitaxel dose reduced to 20 mg/m2. Administration of cabazitaxel to patients with mild hepatic impairment should be undertaken with caution and close monitoring of safety;
(b) In patients with moderate hepatic impairment (total bilirubin >1.5 to ≤ 3.0 x ULN), the maximum tolerated dose (MTD) is 15 mg/m². If the treatment is envisaged in patients with moderate hepatic impairment the dose of cabazitaxel should not exceed 15 mg/m². However, limited efficacy data are available at this dose;
(c) Cabazitaxel should not be given to patients with severe hepatic impairment (total bilirubin >3 x ULN.

The present invention also relates to an article of manufacture comprising:
a) a packaging material;
b) the compound of formula (I) in the form of an acetone solvate; and
c) a label or package insert contained within the packaging material indicating that hepatic impairment can occur.

The article of manufacture as defined above may comprise a label or package insert contained within the packaging material indicating at least one message selected from the group consisting of:
(a) the reduced and recommended dose for patients with mild hepatic impairment is 20 mg/m².
(b) the reduced and maximum tolerated dose for patients with moderate hepatic impairment is 15 mg/m².
(c) the administration of cabazitaxel in patients with mild hepatic impairment may have to be stopped.
(d) the administration of cabazitaxel in patients with severe hepatic impairment is contraindicated.

The article of manufacture as defined above may comprise a label or package insert contained within the packaging material indicating at least one message selected from the group consisting of:
(a) Patients with mild hepatic impairment (total bilirubin >1 to 1.5 x Upper Limit of Normal (ULN) or AST >1.5 x ULN), should have cabazitaxel dose reduced to 20 mg/m2. Administration of cabazitaxel to patients with mild hepatic impairment should be undertaken with caution and close monitoring of safety;
(b) Limited efficacy data for cabazitaxel at 15 mg/m 2, the maximum tolerated dose in patients with moderate hepatic impairment (total bilirubin >1.5 to < 3 x ULN) are available to recommend this dose in this population;
(c) cabazitaxel should not be given to patients with severe hepatic impairment (total bilirubin >3 xULN).

The article of manufacture as defined above may comprise a label or package insert contained within the packaging material indicating at least one message selected from the group consisting of:
(a) Patients with mild hepatic impairment (total bilirubin >1 to ≤1.5 x Upper Limit of Normal (ULN) or AST >1.5 x ULN), should have cabazitaxel dose reduced to 20 mg/m². Administration of cabazitaxel to patients with mild hepatic impairment should be undertaken with caution and close monitoring of safety;
(b) In patients with moderate hepatic impairment (total bilirubin >1.5 to ≤ 3.0 x ULN), the maximum tolerated dose (MTD) is 15 mg/m². If the treatment is envisaged in patients with moderate hepatic impairment the dose of cabazitaxel should not exceed 15 mg/m². However, limited efficacy data are available at this dose;
(c) Cabazitaxel should not be given to patients with severe hepatic impairment (total bilirubin >3 x ULN.

The present invention also relates to a package comprising the compound of formula (I) as defined above and a label, said label comprising one or more messages that:
a) the reduced and recommended dose for patients with mild hepatic impairment is 20 mg/m².
(b) the reduced and maximum tolerated dose for patients with moderate hepatic impairment is 15 mg/m².
(c) the administration of cabazitaxel in patients with mild hepatic impairment may have to be stopped.
(d) the administration of cabazitaxel in patients with severe hepatic impairment is contraindicated.

The present invention also relates to a package comprising the compound of formula (I) as defined above and a label, said label comprising one or more messages that:
(a) Patients with mild hepatic impairment (total bilirubin >1 to 1.5 x Upper Limit of Normal (ULN) or AST >1.5 x ULN), should have cabazitaxel dose reduced to 20 mg/m2. Administration of cabazitaxel to patients with mild hepatic impairment should be undertaken with caution and close monitoring of safety;
(b) Limited efficacy data for cabazitaxel at 15 mg/m², the maximum tolerated dose in patients with moderate hepatic impairment (total bilirubin >1.5 to < 3 x ULN) are available to recommend this dose in this population;
(c) cabazitaxel should not be given to patients with severe hepatic impairment (total bilirubin >3 xULN).

The present invention also relates to a package comprising the compound of formula (I) as defined above and a label, said label comprising one or more messages that:
(a) Patients with mild hepatic impairment (total bilirubin >1 to ≤1.5 x Upper Limit of Normal (ULN) or AST >1.5 x ULN), should have cabazitaxel dose reduced to 20 mg/m². Administration of cabazitaxel to patients with mild hepatic impairment should be undertaken with caution and close monitoring of safety;
(b) In patients with moderate hepatic impairment (total bilirubin >1.5 to ≤ 3.0 x ULN), the maximum tolerated dose (MTD) is 15 mg/m². If the treatment is envisaged in patients with moderate hepatic impairment the dose of cabazitaxel should not exceed 15 mg/m². However, limited efficacy data are available at this dose;
(c) Cabazitaxel should not be given to patients with severe hepatic impairment (total bilirubin >3 x ULN.

In the package of the invention, the compound of formula (I) may be in the form of an acetone solvate.

### EXAMPLE

Study Title: Phase I safety and pharmacokinetic study of XRP6258 (cabazitaxel) in advanced solid tumor patients with varying degrees of hepatic impairment (POP6792)

### OBJECTIVES

- To determine the maximum tolerated dose (MTD) and safety of cabazitaxel administered to advanced solid tumor patients with varying degrees of hepatic impairment.
- To determine the pharmacokinetics (PK) of cabazitaxel in patients with varying degrees of hepatic impairment.
- To correlate PK variables with pharmacodynamic (PD) safety parameters in order to guide prescribers with regard to dosing in this patient population.

### ENDPOINTS AND STATISTICAL METHODS

- The primary endpoint was the occurrence of DLTs during Cycle 1.
- The secondary endpoints were:
   - Treatment-emergent adverse events (TEAEs);
   - Laboratory abnormalities; and,
   - Pharmacokinetic parameters (Cmax, Ceoi, tmax, tlast, AUClast, AUCO-24, AUCO-72, t1/2z, AUC, CL and Vss).
- The effect of the degree of hepatic impairment on cabazitaxel PK parameters was evaluated according to a linear mixed effects model (Log [parameter] = Cohort + Error) with a fixed term for Cohort, fit by estimated generalized least squares (GLS) using SAS PROC MIXED. Estimate and 90% confidence interval (CI) for the geometric mean ratio of each cohort versus Cohort 1 and of Cohorts 3 and 4 versus Cohort 2 were provided for CL, CL/BSA, and exposure parameters (Cmax, AUC, AUClast, AUC0-24 and AUC0-72) normalized to actual cabazitaxel dose in mg/m².

### STUDY POPULATION

| | |
|---|---|
| Ages Eligible for Study: | 18 Years and older |
| Genders Eligible for Study: | Both |
| Accepts Healthy Volunteers: | No |

### Inclusion criteria:

- Patients with a diagnosis of advanced, measurable or non-measurable, non-hematological cancer who have varying degrees of hepatic impairment. The cancer must be one that is either refractory to standard therapy or for which no standard therapy exists.

### Exclusion criteria:

- Eastern Cooperative Oncology Group (ECOG) performance status (PS) >2
- Life expectancy <3 months
- Need for a major surgical procedure or radiation therapy during the study
- Evidence of another active malignancy
- Prior chemotherapy, other investigational drug, biological therapy, targeted non-cytotoxic therapy and radiotherapy within 3 weeks prior to registration
- Patients with known history of Gilbert's syndrome
- Prior treatment with Cabazitaxel and a history of severe (Grade ≥3) hypersensitivity to taxanes, polysorbate-80, or to compounds with similar chemical structures

### TRIAL DESIGN

- The study was designed as an open-label, dose-escalation, multi-center study of cabazitaxel administered as a 1-hour intravenous (IV) infusion every 3 weeks in cancer patients with varying degrees of hepatic impairment. Patients were enrolled in 4 cohorts based on hepatic function at baseline graded by total bilirubin and aspartate aminotransferase (AST):
   - Cohort 1: normal hepatic function (total bilirubin supper limit of normal [ULN]; aspartate aminotransferase [AST] ≤ULN),
   - Cohort 2: mild hepatic impairment (total bilirubin >1 to ≤1.5xULN or AST
      >1.5xULN),
   - Cohort 3: moderate hepatic impairment (total bilirubin >1.5x to ≤3.0xULN; AST=any),
   - Cohort 4: severe hepatic impairment (total bilirubin >3.0x - 10xULN; AST=any).
   All liver function tests must be completed within 48 hours prior to start of treatment, and reviewed by the investigator or his designee before treatment administration.

### • Dose regimen:

Cabazitaxel is administered on Day 1 of each 3-week cycle. Patients received cabazitaxel administered by IV infusion over 1 hour at the dose specified for each cohort. The actual amount of cabazitaxel administered was adjusted at each cycle based on body surface area (BSA) to a maximum BSA of 2.1 m² (i.e., patients with a BSA over 2.1 m2 will receive the same amount of cabazitaxel as those with a BSA of 2.1 m²).

Required IV premedication included: antihistamine (dexchlorpheniramine 5 mg, diphenhydramine 25 mg or other antihistamines); steroid (dexamethasone 8 mg or equivalent steroid). H2 antagonist (ranitidine or other H2 antagonist with the exception of cimetidine).

These premedications were administered by IV infusion, at least 30 minutes prior to each dose of cabazitaxel.

New cycles of therapy did not begin until the absolute neutrophil count (ANC) ≥1,500/mm3, platelet count ≥75,000 /mm3, hemoglobin ≥ 9.0 g/dL, INR ≤1.3, serum creatinine ≤1.5 mg/dL or ≤1.5 ULN, and non-hematologic toxicities (except alopecia, asthenia, local reactions, and other toxicities that are uncomfortable but do not cause serious morbidity to patients) have recovered to either Grade ≤1 or baseline grades.

A maximum of 2 weeks treatment delay was allowed between treatment cycles. Patients discontinued study treatment if treatment delay exceeds 2 weeks.

Patients continued on treatment until they experience unacceptable toxicities/AEs, disease progression, withdraw their consent, the investigator decided to withdraw the patient, or study cutoff date, whichever occurs first.

The cutoff date for full data collection was when the last patient has completed cycle 1 treatment and the subsequent 30 days follow up. However, if patients were still receiving treatment after the cutoff date, SAEs and investigational medicinal product (IMP) administration continued to be captured and communicated to the sponsor.
- The starting dose of cabazitaxel was:
   - Cohort 1: 25 mg/m²,
   - Cohort 2: 20 mg/m²,
   - Cohort 3: 10 mg/m²,
   - Cohort 4: 10 mg/m².
- As planned in protocol, there was no dose escalation for Cohort 1. For Cohorts 2, 3 and 4, dose-escalation was based on dose-limiting toxicities (DLTs) during Cycle 1; real time PK information was used along with safety information. The maximum administered dose (MAD) was to be reached at the dose level when at least 2 patients develop a DLT during Cycle 1. The MTD was defined as the highest dose at which no more than 0 of the first 3 patients, or 1 of 6 patients, experience a DLT during Cycle 1, to a maximum dose of 25 mg/m2. The MTD was one dose level below the MAD.

Cohort 3 has been initiated after at least 3 patients of Cohort 2 completed the first cycle of treatment with no dose-limiting toxicities (DLTs) being observed. Similarly, Cohort 4 began after 3 patients complete the first cycle of Cohort 3, providing no DLT has occurred.

### Dose escalation schedule for Cohort 2:

| Dose levels | Cabazitaxel, mg/m² |
|---|---|
| -1 | 15 |
| 0 (Starting dose) | 20 |
| +1 | 25 |

### Dose escalation schedule for Cohort 3:

| Dose levels | Cabazitaxel, mg/m² |
|---|---|
| 0 (Starting dose) | 10 |
| +1 | 15 |
| +2 | 20 |
| +3 | 25 |

### Dose escalation schedule for Cohort 4:

The starting dose in Cohort 4 could be 5 or 10 mg/m², was decided by the study committee based on the safety and PK information from the first 3 patients in Cohort 3.

| Dose levels | Cabazitaxel, mg/m² | Cabazitaxel, mg/m² |
|---|---|---|
| 0 (Starting dose) | 5 | 10 |
| +1 | 10 | 15 |
| +2 | 15 | 20 |
| +3 | 20 | 25 |

### Dose escalation criteria:

For each cohort, the dose escalation criteria as described in the table below had to be met at each dose level following Cycle 1 in order to enroll and treat additional patients at the next dose level. For Cohorts 2, 3 and 4, a 1 week gap was required between the treatment of each patient for the first 3 patients in each dose level to allow safety evaluation.

**Cabazitaxel dose-escalation decision rules for Cohorts 2, 3 and 4:**

| Patients with Cycle 1 DLT at a given dose level | Dose-escalation decision rule |
|---|---|
| 0 of the first 3 patients | Escalate dose level and enter at least 3 patients at next dose level (Cohorts 2 through 4) or at the same dose level (Cohort 1) |
| 1 out of the first 3 patients | Enter up to 3 additional patients at this dose level. |
| | If 0 of the 3 additional patients experience a DLT, then proceed to the next higher dose level, to a maximum dose of 25 mg/m² |
| | If 1 or more of the 3 additional patients experience a DLT, then dose escalation will be stopped. Decrease dose to the next available lower dose level. If a lower dose is not available, enrollment will be stopped for a given cohort. |
| 2 of the first 3 patients | Decrease dose to the next available lower dose level. If a lower dose is not available, enrollment will be stopped for a given cohort |

### Dose-Limiting Toxicities (DLT) and Maximum Tolerated Dose (MTD):

To be considered a DLT, the clinical adverse event (AE) or laboratory abnormality should be drug-related as assessed by the investigator.

Liver DLTs in patients with hepatic dysfunction (Cohorts 2, 3 and 4) were defined as an increase in total and direct bilirubin and/or transaminase levels to 3 times the baseline value. In addition, a treatment delay due to cabazitaxel related toxicity of 2 weeks between cycles was considered a DLT.

All other DLTs were defined [according to the National Cancer Institute Common Terminology Criteria for Adverse Events (NCI CTCAE) Version 4.0 grading scale] during the first treatment cycle, as follows:

### Non-hematological toxicity Grade 3 or 4 except:

- Grade 3 fever without documented infection
- Grade 3 nausea and vomiting in the absence of effective maximal antiemetic therapy
- Grade 3 mucositis/stomatitis in the absence of effective symptomatic treatment
- Grade 3 fatigue
- Grade 3 anorexia
- Grade 3 AST/ALT or bilirubin elevation that returns to baseline prior to next treatment cycle (Cohort 1 only)
- Grade 3 hypersensitivity reaction in the absence of required premedication
- Peripheral neuropathy Grade 3 that returns to Grade 2 or less at the initiation of next treatment cycle

Hematological toxicity defined as:
- Febrile neutropenia: fever (of unknown origin without clinically or microbiologically documented infection) ≥ 38.5°C with neutropenia Grade 3 or 4
- Neutropenia Grade 4 lasting >7 days
- Thrombocytopenia Grade 4

If 1 DLT occurs in the first cycle, 3 additional patients were included at the same dose level. The MAD of Cohort 2, 3 and 4 was reached at the dose level when at least 2 patients develop a DLT during the first cycle.

Patients who were not treated, or were withdrawn during the first 3 weeks of treatment, for reasons other than DLT and before the MAD is determined, were replaced.

Maximal Tolerated Dose: The MTD was defined as the highest dose at which 0 of the first 3 patients, or 1 of 6 patients experienced a DLT during the first cycle of cabazitaxel to a maximum dose of 25 mg/m². The MTD was one dose below the MAD. If the MAD reached at 5 or 10 mg/m² (Cohorts 3 and 4), then no patients were further enrolled as the MTD could not be established. There was no dose recommendation for such patient population.

Prophylactic and therapeutic use of hematopoietic growth factors was not permitted during the first cycle of study treatment unless a hematological DLT is encountered.

### • Safety Data

Vital signs, medical history, physical examinations, Eastern Cooperative Oncology Group (ECOG) performance status (PS), electrocardiogram (ECG), and laboratory safety tests (including hematology, serum chemistries, and urinalysis) were obtained prior to study drug administration and at designated intervals throughout the study. Treatment-emergent AEs (TEAEs) were collected during the study and up to 30 days after the end of study treatment. Any serious adverse events (SAEs) considered related to the study medication were collected regardless of when they occur. Adverse events were graded according to the NCI CTCAE v. 4.0).

However, if patients were still receiving treatment after the cutoff date, SAEs and IMP administration continued to be captured.
- Blood samples for the determination of cabazitaxel (total) plasma concentrations were to be collected from all patients at the following time points in Cycle 1: immediately before the start of infusion, 5 minutes (min) before the end of infusion, then 5 min, 15 min, 30 min, 1 hour (h), 2 h, 3 h, 5 h, 7 h and 10 h after the end of infusion, and then 24 h (Day 2), 48 h (Day 3), 72 h (Day 4), 96 h (Day 5), 168 h (Day 8) and 216 h (Day 10) after the start of infusion. Total cabazitaxel concentrations in plasma were determined using a validated liquid chromatography with tandem mass spectrometry method (LC-MS/MS) with a lower limit of quantification (LLOQ) of 1 ng/mL.

Real time PK information has been used to inform the dose selection in the next dose level for Cohort 3 and 4 and in the starting dose of Cohort 4 along with safety information.

### RESULTS

- A total of 43 patients were enrolled and treated with cabazitaxel. Six (6) patients were treated in Cohort 1 (at 25 mg/m²). The MTD in Cohort 2 was 20 mg/m² and a total of 12 patients were treated at this dose level. The MTD in Cohort 3 was 15 mg/m² and a total of 7 patients were treated at this dose level. Cohort 4 was prematurely closed in agreement with the FDA after a total of 7 patients were treated; the MAD and MTD were not determined. The safety population comprised all 43 enrolled patients, 38 patients were evaluable for DLT assessment, and the PK population comprised 38 patients.
- All patients had discontinued study treatment by the cut-off date, with the exception of 1 patient in Cohort 2 at 20 mg/m². The most frequent reason for study treatment discontinuation was disease progression overall (28/43 patients [65.1%]) and at the MTD in Cohorts 2 and 3 (8/12 patients [66.7%] and 4/7 patients [57.1%], respectively).
- Most of the patients in the safety population were Caucasian/White (34/43 patients [79.1%]). Male and female patients were approximately equally represented. The median age was 60.0 years (range: 18:79). The ECOG performance status was 1 in most of the patients (35/43 patients [81.4%]). The most frequent primary tumor sites were colon and liver (8/43 patients [18.6%] each). The disease stage at entry in the study was metastatic in most of the patients (39/43 patients [90.7%]). Prior anticancer therapies were given as follows: drug therapy in all patients, radiotherapy in 20/43 patients (46.5%) and surgery in 15/43 patients (34.9%).
- The median [range] number of study treatment cycles administered per patient in Cohorts 1, 2 at the MTD and 3 at the MTD was 3.0 [1:4], 2.0 [1:31] and 2.0 [1:3], respectively. The median [range] relative dose intensity (RDI) was 0.991 [0.63:1.01], 0.971 [0.84:1.02] and 0.895 [0.72:1.01], respectively. The number [%] of patients with at least 1 cycle administered at a reduced dose of cabazitaxel in Cohorts 1, 2 (at the MTD) and 3 (at the MTD) was 2/6 [33.3%], 3/12 [25.0%] and 3/7 [42.9%], respectively.
- In Cohort 1, DLTs were reported in Cycle 1 in 3/4 DLT-evaluable patients (75.0%); these were Gr 4 febrile neutropenia (1 Pt), Gr 4 neutropenia (1 Pt), Gr 4 atrial fibrillation and Gr 4 hypotension (1 Pt). The MTD in Cohort 2 was 20 mg/m2; the MAD was 25 mg/m2. At the MTD, 3/11 DLT-evaluable patients (27.3%) experienced a DLT during Cycle 1 (Gr 4 Candida sepsis, Gr 4 febrile neutropenia and Gr 4 platelet count decreased in 1 Pt; Gr 4 febrile neutropenia and Gr 3 diarrhea in 1 Pt; and, Gr 4 neutropenia in 1 Pt). The MTD in Cohort 3 was 15 mg/m2; the MAD was 20 mg/m2. At the MTD, 1/6 DLT-evaluable patients (16.7%) experienced a DLT during Cycle 1 (Gr 3 hepatic encephalopathy).
- The MTD and MAD in Cohort 4 were not determined due to premature discontinuation of this cohort following the death of the first patient treated at the 20 mg/m2 dose level due to AEs (including DLTs of Gr 3 dehydration, Gr 4 septic shock, Gr 4 tumor lysis syndrome and Gr 3 urinary tract infection) and disease progression. At the lower dose levels evaluated (10 and 15 mg/m²), none of the 6 evaluable patients experienced a DLT during Cycle 1.
- The most frequent non-hematological study drug-related TEAEs were fatigue in Cohort 1 (5/6 patients [83.3%]), and diarrhea in Cohort 2 at the MTD and in Cohort 3 at the MTD (5/12 patients [41.7%] and 3/7 patients [42.9%], respectively). Study drug-related febrile neutropenia was reported in 1/6 patients (16.7%) in Cohort 1, 3/12 patients (25.0%) in Cohort 2 at the MTD, and 1/7 patients (14.3%) in Cohort 3 at the MTD. No patient experienced febrile neutropenia in Cohort 4.
- TEAEs leading to cabazitaxel dose reduction in Cohort 1, Cohort 2 at the MTD, and Cohort 3 at the MTD (2/6 patients [33.3%], 2/12 patients [11.1 %] and 2/7 patients [16.7%], respectively) were neutropenia (Gr ≥3)and/or febrile neutropenia (Gr >3). No

TEAEs leading to permanent study treatment discontinuation were reported in more than 1 patient in any Cohort/dose level.
- The most frequent Gr ≥3 on-treatment hematology laboratory abnormalities in Cohort 1, Cohort 2 at the MTD, and Cohort 3 at the MTD were neutrophil count decreased and white blood cell count decreased (5/6 patients [83.3%] each, 9/12 patients [75.0%] each and 4/7 patients [57.1%] each, respectively).
- The study drug-related serious TEAEs reported in more than 1 patient in any Cohort/dose level were neutropenia (Gr ≥3)(2/6 patients [33.3%] in Cohort 1 and 2/6 patients [33.3%] in Cohort 2/25 mg/m²) and febrile neutropenia (3/12 patients [25.0%] in Cohort 2 at the MTD). Twenty-three (23) patients (53.5%) died by the cut-off date; the most frequent reason for death was disease progression (21/43 patients [48.8%]). One patient in Cohort 2/20 mg/m² died due to Gr 4 Candida sepsis related to study drug. One patient in Cohort 4/20 mg/m² died due to a combination of shock, acute renal failure, tumor lysis syndrome (all related to study drug), acute respiratory failure (not related to study drug), and disease progression.
- The patients in Cohort 3/10 mg/m² had an aberrant PK behavior with a mean CL/BSA (517 L/h/m2) approximately 20-fold higher than that for the other Cohort 3 patients (15 and 20 mg/m2: 30.5 L/h/m2). These patients were excluded from the statistical analysis of the effect of hepatic impairment on cabazitaxel PK.
- CL/BSA estimate in Cohort 1 (13.4 L/h/m2; 90% Cl: 8.6-20.8) was in the very low range of the typical cabazitaxel clearance determined by population PK analysis (POH0124) (26.4 L/h/m2, CV=38.8%, n=170). Because of these low values of CL/BSA in Cohort 1, this made an assessment of the effect of hepatic impairment on cabazitaxel PK by comparison with Cohort 1 impractical.
- CL/BSA estimate in Cohort 2 (23.5 L/h/m2; 90% Cl: 17.6-31.4) was in the range of those typically observed in previous studies (POH0124) suggesting that mild hepatic impairment has no effect on cabazitaxel PK parameters. While a comparison of Cohort 2 to Cohort 1 showed a 75% increase of CL/BSA in Cohort 2 (ratio=1.75; 90% Cl: 1.04-2.96), it is
   likely this is due to a lower than normal CL/BSA value for Cohort 1.
- A comparison of Cohorts 3 and 4 to Cohort 2 showed a 19% increase of CL/BSA in Cohort 3 (ratio=1.19; 90% Cl: 0.74-1.91) while a 23% decrease of CL/BSA (ratio=0.77; 90% Cl: 0.39-1.53) was observed in Cohort 4. The opposite trend was observed for AUC parameters; the highest magnitude effect was observed on AUClast/dose, which showed a 14% decrease in Cohort 3 (ratio=0.86; 90% Cl: 0.50-1.46) and a 17% increase in Cohort 4 (ratio=1.17; 90% Cl: 0.63-2.14).
- A sensitivity analysis performed after exclusion of patients with an erratic PK profile provided consistent findings, with a 6% decrease of CL/BSA in Cohort 3 (ratio=0.94; 90% Cl: 0.64-1.38) and a 39% decrease of CL/BSA in Cohort 4 (ratio=0.61; 90% Cl: 0.36-1.05).

### CONCLUSION

- The MTD of cabazitaxel administered as a 1-hour IV infusion every 3 weeks in advanced solid tumor patients with mild hepatic impairment (total bilirubin >1 to ≤1.5xULN or AST>1.5xULN) was 20 mg/m2. The MTD in patients with moderate hepatic impairment (total bilirubin >1.5x to ≤3.0xULN; AST=any) was 15 mg/m2.
- The MTD in patients with severe hepatic impairment (total bilirubin >3.0x - 10xULN; AST=any) was not determined due to premature discontinuation of this cohort following the death of the first patient treated at the 20 mg/m2 dose level due to AEs (including DLTs) and disease progression. At the lower dose levels evaluated (10 and 15 mg/m²), none of the 6 evaluable patients experienced a DLT during Cycle 1.
- The safety profile of cabazitaxel at the MTD in patients with mild or moderate hepatic impairment was generally similar to that observed at 25 mg/m² in patients with normal hepatic function. Overall, the safety profile of cabazitaxel in POP6792 was consistent with the known safety profile of cabazitaxel; no new safety issues were identified.
- Cabazitaxel CL/BSA estimates were 13.4 L/h/m2 in patients with normal hepatic function, and 23.5, 27.9 and 18.1 L/h/m2 in patients with mild, moderate or severe hepatic impairment, respectively. CL/BSA in patients with normal hepatic function was in the very low range of the typical CL/BSA determined from the historical patient database analysis in POH0124 (26.4 L/h/m2, CV=38.8%, n=170). Therefore, there is no evidence that mild or moderate hepatic impairment results in a substantial decline in cabazitaxel clearance.

## Claims

1. Compound having the following formula (I): 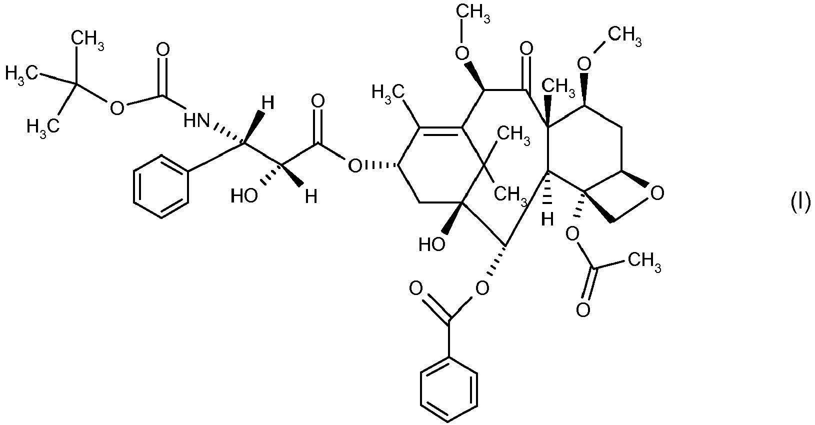 which may be in base form or in the form of a hydrate or a solvate,
for its use as a medicament in the treatment of cancer in patients with hepatic impairment where the administered standard dose of 25 mg/m² of the compound is reduced or where the administration of the compound is stopped or to be avoided.

2. Compound for the use according to claim 1, wherein the hepatic impairment is mild, moderate or severe.

3. Compound for the use according to claim 1 or 2, wherein the cancer to be treated is a solid tumor.

4. Compound for the use according to claim 3, wherein the cancer to be treated is a castration resistant or hormone-refractory metastatic prostate cancer.

5. Compound for the use according to any one of claims 1 to 4, wherein the compound of formula (I) is in the form of an acetone solvate.

6. Compound for the use according to any one of claims 1 to 5, wherein the compound is administered by intravenous infusion at a dose of between 15 and 25 mg/m².

7. Compound for the use according to any one of claims 1 to 6 in patients with mild hepatic impairment, wherein the compound is administered at a dose of 20 mg/m².

8. Compound for the use according to any one of claims 1 to 6 in patients with moderate hepatic impairment, wherein the compound is administered at a dose of 15 mg/m².

9. Compound for the use according to any one of claims 1 to 6 in patients with moderate hepatic impairment, wherein the administration of the compound is stopped.

10. Compound for the use according to any one of claims 1 to 6 in patients with severe hepatic impairment, wherein the administration of the compound is contraindicated.

11. An article of manufacture comprising:
a) a packaging material;
b) the compound of formula (I) in the form of an acetone solvate; and
c) a label or package insert contained within the packaging material indicating at least one message selected from the group consisting of:
(1) the reduced and recommended dose for patients with mild hepatic impairment is 20 mg/m².
(2) the reduced and maximum tolerated dose for patients with moderate hepatic impairment is 15 mg/m².
(3) the administration of cabazitaxel in patients with mild hepatic impairment may have to be stopped.
(4) the administration of cabazitaxel in patients with severe hepatic impairment is contraindicated.

12. A package comprising the compound of formula (I) of claim 1 and a label, said label comprising one or more messages that:
a) the reduced and recommended dose for patients with mild hepatic impairment is 20 mg/m².
b) the reduced and maximum tolerated dose for patients with moderate hepatic impairment is 15 mg/m².
c) the administration of cabazitaxel in patients with mild hepatic impairment may have to be stopped.
d) the administration of cabazitaxel in patients with severe hepatic impairment is contraindicated.

13. A method of treating cancer in a patient in need thereof, comprising administering a reduced dose of cabazitaxel, in base form or in a hydrate or solvate thereof, to said patient, wherein said patient has hepatic impairment, and wherein said reduced dose is less than the standard dose of 25 mg/m² of cabazitaxel.

14. The method according to claim 13, wherein the hepatic impairment is mild, moderate or severe.

15. The method according to claim 13 or claim 14, wherein the cancer to be treated is a solid tumor.

16. The method according to claim 15, wherein the cancer to be treated is a castration resistant or hormone-refractory metastatic prostate cancer.

17. The method according to claim 16, wherein the compound is administered in combination with a corticoid.

18. The method according to any one of claims 13 to 17, wherein the compound of formula (I) is in the form of an acetone solvate.

19. The method according to any one of claims 13 to 18, wherein the compound is administered by intravenous infusion at a dose of 15 to 20 mg/m².

20. The method according to any one of claims 13 to 19, wherein the compound is administered at a dose of 15 mg/m², and wherein the patient has moderate hepatic impairment.
